# EUROPÄISCHE PATENTSCHRIFT

(11) **EP 0 743 298 B1**
(45) Veröffentlichungstag und Bekanntmachung des Hinweises auf die Patenterteilung: **14.10.1998**
(21) Anmeldenummer: 96107426.7
(22) Anmeldetag: 10.05.1996
(51) Int. Cl.: C07C 68/02, C07C 69/96

(54) **Verfahren zur Herstellung von Chlorameisensäurearylestern**
Process for the preparation of aryl chloroformates
Procédé de préparation de chloroformiates d'aryle

(30) Priorität: 19.05.1995 DE 19518473
(43) Veröffentlichungstag der Anmeldung: 20.11.1996
(73) Patentinhaber: BASF AKTIENGESELLSCHAFT, 67056 Ludwigshafen (DE)
(72) Erfinder: Kahl, Thomas-Michael, 67354 Römerberg (DE); Wettling, Thomas, 67117 Limburgerhof (DE)

(56) Entgegenhaltungen:
- EP-A- 0 080 913
- DE-A- 2 131 555
- US-A- 3 334 128

## Beschreibung

Die vorliegende Erfindung betrifft ein Verfahren zur Herstellung von Chlorameisensäurearylestern durch Umsetzung von Phenolen mit Phosgen in Anwesenheit eines cyclischen Harnstoffes.

Die Herstellung von Chlorameisensäurearylestern durch Umsetzung von Phenolen mit Phosgen in Anwesenheit von unterstöchiometrischen Mengen eines Katalysators bei erhöhten Drücken ist für die Katalysatoren Dimethylformamid aus US-A-3 211 774, polymere Verbindungen mit freien sekundären oder tertiären Aminogruppen aus US-A-3 211 775 und Amine sowie Ammoniumsalze aus US-A-3 211 776 bekannt.

Umsetzungen von Phosgen unter Druck sind verfahrenstechnisch und sicherheitstechnisch schwer zu beherrschen und erfordern einen hohen technischen Aufwand.

Als unterstöchiometrisch eingesetzte Katalysatoren für Umsetzungen bei Normaldruck sind aliphatische Säureamide und acyclische Harnstoffe aus DE-A-21 31 555, Ammoniumsalze aus DE-A-30 00 524, Phosphine, Phosphinoxide und Phosphoniumsalze aus DE-A-30 19 526 sowie Phosphite aus DE-A-41 37 640 bekannt.

Diese Katalysatoren haben den Nachteil der mäßigen Umsatzgeschwindigkeiten von Phosgen/Phenol und verstärkter Bildung von Arylchloriden während Reaktion oder der Destillation, meist verbunden mit der Desaktivierung des Katalysators. Ferner ist die Entsorgung phosphorhaltiger Destillationsrückstände durch die Bildung von Phosphorsäure bei der Verbrennung erschwert.

Der vorliegenden Erfindung lag daher die Aufgabe zurgrunde, den zuvor genannten Nachteilen abzuhelfen, insbesondere ein Verfahren zu finden, das mit phosphorfreien Katalysatoren zu hohen Raum-Zeit-Ausbeuten führt.

Demgemäß wurde ein neues und verbessertes Verfahren zur Herstellung von Chlorameisensäurearylestern der allgemeinen Formel I 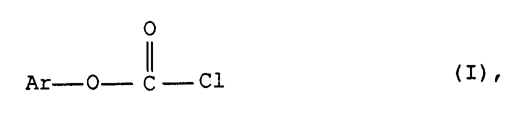 in der
- Ar: ein gegebenenfalls ein- bis fünffach durch C₁- bis C₆-Alkyl, C₂- bis C₆-Alkenyl, C₃- bis C₈-Cycloalkyl, C₄- bis C₁₀-Cycloalkyl-alkyl, C₁- bis C₆-Halogenalkyl, C₁- bis C₆-Alkoxy, C₁- bis C₄-Alkylthio, Halogen, Cyano, C₂- bis C₆-Alkylcarbonyloxy, Formyl, C₂- bis C₆-Dialkylamino, Aryl, Aryloxy, Arylthio, Aroyl, C₇- bis C₁₀-Aralkyl, C₇- bis C₁₀-Aralkoxy, Arylsulfonyl und/oder C₇- bis C₁₀-Aralkylthio und/oder ein- bis zweifach durch Chlorformyl und/oder Nitro substituiertes Aryl
bedeutet, durch Umsetzung von Phosgen und einem Phenol der allgemeinen Formel II

Ar―OH II

, in der Ar die obengenannten Bedeutungen hat, in Gegenwart einer stickstoffenthaltenden Verbindung bei Temperaturen von 60 bis 180°C und Drücken von 0,01 bis 5 bar gefunden, welches dadurch gekennzeichnet ist, daß man als stickstoffenthaltende Verbindung einen cyclischen Harnstoff der allgemeinen Formel III 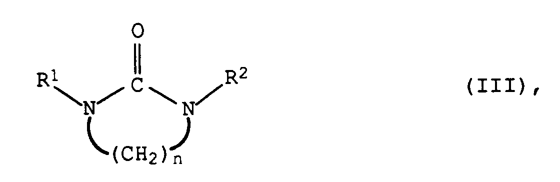 in der R¹ und R² für C₁- bis C₄-Alkyl und n für 2 oder 3 stehen, einsetzt.

Das erfindungsgemäße Verfahren läßt sich wie folgt durchführen:

Das Phenol II kann kontinuierlich oder diskontinuierlich, bevorzugt kontinuierlich, in Gegenwart eines cyclischen Harnstoffes III als Katalysator bei Temperaturen von 60 bis 180°C, bevorzugt 80 bis 160°C, besonders bevorzugt 100 bis 140°C und Drücken von 0,01 bis 50 bar, bevorzugt 0,5 bis 5 bar, besonders bevorzugt 0,9 bis 1 bar, insbesonders Normaldruck (Atmosphärendruck), bevorzugt in Flüssigfahrweise, besonders bevorzugt in homogener flüssiger Phase wie in einem inerten Lösungsmittel oder in der Schmelze des Phenols II, mit Phosgen umgesetzt werden.

Das bevorzugte kontinuierliche Verfahren kann beispielsweise im Reaktionsrohr, in einer Rührkesselkaskade, in einem Schlaufenreaktor oder in einer Gegenstromkolonne durchgeführt werden.

Das Molverhältnis von cyclischem Harnstoff III zum Phenol II liegt in der Regel bei 0,001:1 bis 0,2:1, bevorzugt 0,002:1 bis 0,1:1, besonders bevorzugt 0,005:1 bis 0,05:1.

Das Molverhältnis von Phosgen zum Phenol II beträgt in der Regel 0,5:1 bis 50:1, bevorzugt 1:1 bis 2:1, besonders bevorzugt 1:1 bis 1,5:1, insbesondere 1:1 bis 1,2:1.

Als inerte Lösungsmittel eignen sich beispielsweise aliphatische und aromatische Kohlenwasserstoffe wie Pentan, Hexan, Cyclohexan, Toluol, Xylol oder Benzol, halogenierte Kohlenwasserstoffe wie Trichlorethan, Chlorbenzol oder Dichlorbenzol oder Ester wie Ethylacetat oder Butylacetat oder das Chlorformiat des entsprechenden Phenols.

In bevorzugter Weise wird in der Regel entweder ohne Lösungsmittel nur in der Schmelze des Phenols, oder unter Verwendung der z.B. durch Phosgenierung der beschriebenen Phenole entstehenden Chlorformiate als Lösungsmittel gearbeitet. Jedoch kann das Arbeiten im Lösungsmittel dann vorteilhaft sein, wenn der Schmelzpunkt des einzusetzenden Phenols oder des entsprechenden Chlorformiats oberhalb der angestrebten Reaktionstemperatur liegt und das Phenol, zumindest zu Beginn der Reaktion, ohne Gegenwart des Lösungsmittels nur langsam mit dem Phosgen reagieren würde. Die Gegenwart eines Lösungsmittels kann auch zur Beherrschung und Abführung der Reaktionswärme der exothermen Reaktion günstig sein.

Die Aufarbeitung des Reaktionsgemisches kann z.B. durch Destillation erfolgen. Der Destillationsrückstand enthält in der Regel den cyclischen Harnstoff III, der für eine weitere Umsetzung des erfindungsgemäßen Verfahrens wieder eingesetzt werden kann.

Die Substituenten Ar, R¹, R² und der Index n in den Verbindungen I, II und III haben folgende Bedeutungen:
- Ar: - Aryl, wie Phenyl, 1-Naphthyl, 2-Naphthyl, 1-Anthryl, 2-Anthryl und 9-Anthryl, bevorzugt Phenyl,
- ein gegebenenfalls ein- bis fünffach durch einen der folgenden Reste substituiertes Aryl,
- C₁- bis C₆-Alkyl, bevorzugt C₁- bis C₄-Alkyl wie Methyl, Ethyl, n-Propyl, iso-Propyl, n-Butyl, iso-Butyl, sec.-Butyl, tert.-Butyl,
- C₂- bis C₆-Alkenyl, bevorzugt Vinyl und Allyl,
- C₃- bis C₈-Cycloalkyl bevorzugt C₄- bis C₆-Cycloalkyl wie Cyclobutyl, Cyclopentyl und Cyclohexyl,
- C₄- bis C₁₀-Cycloalkyl-alkyl, bevorzugt C₆- bis C₈-Cycloalkyl-alkyl wie Cyclopentyl-methyl, Cyclohexyl-methyl, Cyclobutyl-ethyl, Cyclohexyl-ethyl, Cyclopropyl-propyl, Cyclobutyl-propyl, Cyclopentyl-propyl und Cyclohexyl-propyl,
- C₁- bis C₆-Halogenalkyl, bevorzugt C₁- bis C₄-Halogenalkyl, besonders bevorzugt C₁- bis C₄-Fluor und Chloralkyl wie Fluormethyl, Difluormethyl, Trifluormethyl, Chlormethyl, Dichlormethyl, Trichlormethyl, 1-Chlorethyl, 2-Chlorethyl,
- C₁- bis C₆-Alkoxy, bevorzugt C₁-bis C₄-Alkoxy wie Methoxy, Ethoxy, n-Propoxy, iso-Propoxy, n-Butoxy, iso-Butoxy, sec.-Butoxy, tert.-Butoxy,
- C₁- bis C₆-Alkylthio, bevorzugt C₁- bis C₄-Alkylthio wie Methylthio, Ethylthio, n-Propylthio, iso-Propylthio, n-Butylthio, iso-Butylthio, sec.-Butylthio, tert. Butylthio,
- Halogen wie Fluor, Chlor, Brom und Jod, bevorzugt Fluor, Chlor, besonders bevorzugt Chlor,
- Cyano,
- Formyl,
- C₂- bis C₆-Alkylcarbonyloxy, bevorzugt C₂- bis C₄-Alkylcarbonyloxy wie Acetyloxy, Propionyloxy, Butyryloxy,
- C₂- bis C₆-Dialkylamino, bevorzugt Di-C₁-C₃-alkylamino, wie N,N-Dimethylamino, N,N-Diethylamino, N,N-Dipropylamino, N,N-Di-(1-methylethyl)amino, N-Ethyl-N-methylamino, N-Methyl-N-propylamino, N-Methyl-N-(1-methylethyl)amino, N-Ethyl-N-propylamino, N-Ethyl-N-(1-methyl-ethyl)amino, N-(1-Methylethyl)-N-propylamino, vorzugsweise N,N-Dimethylamino, N,N-Diethylamino insbesondere N,N-Dimethylamino,
- Aryl, wie Phenyl, 1-Naphthyl, 2-Naphthyl, 1-Anthryl, 2-Anthryl und 9-Anthryl, o-Tolyl, m-Tolyl und p-Tolyl, bevorzugt Phenyl,
- Aryloxy wie Phenoxy, 1-Naphthoxy, 2-Naphthoxy, 1-Anthroxy, 9-Anthroxy, o-Tolyloxy, m-Tolyloxy und p-Tolyloxy, bevorzugt Phenoxy,
- Arylthio wie Phenylthio, 1-Naphthylthio, 2-Naphthylthio, 1-Anthrylthio, 2-Anthrylthio und 9-Anthrylthio, bevorzugt Phenylthio,
- Aroyl wie Benzoyl, 1-Naphthoyl, 2-Naphthoyl, 1-Anthracencarbonyl, 2-Anthracencarbonyl und 9-Anthracencarbonyl, bevorzugt Benzoyl
- C₇- bis C₁₀-Aralkyl, bevorzugt C₇- bis C₈-Phenylalkyl wie Benzyl, 1-Phenethyl, 2-Phenethyl, besonders bevorzugt Benzyl,
- C₇- bis C₁₀-Aralkoxy, bevorzugt C₇- bis C₈-Phenylalkoxy wie Benzyloxy, 1-Phenethoxy, 2-Phenethoxy,
- Arylsulfonyl wie Phenylsulfonyl, 1-Naphthylsulfonyl, 2-Naphthylsulfonyl, 1-Anthrylsulfonyl, 2-Anthrylsulfonyl und 9-Anthrylsulfonyl, bevorzugt Phenylsulfonyl,
- C₇- bis C₁₀-Aralkylthio bevorzugt C₇- bis C₈-Phenylalkylthio wie Benzylthio, 1-Phenethylthio, 2-Phenethylthio, besonders bevorzugt Benzylthio,
- und/oder ein gegebenenfalls ein- bis zweifach durch einen der folgenden Reste substituiertes Aryl,
- Chlorformyl,
- Nitro,
- sowie im Falle von den Verbindungen II noch zusätzlich ein oder zwei Hydroxyfunktionen
- R₁, R₂: - unabhängig voneinander
- C₁- bis C₄-Alkyl bevorzugt C₁- bis C₂-Alkyl wie Methyl, Ethyl,
- n: - 2 oder 3.

Chlorameisensäurearylester I sind wertvolle Zwischenprodukte, beispielsweise bei der Herstellung von Farbstoffen, wie Sirius-Farbstoffe (DE-A-23 25 088, Ullmanns Enzyklopädie der technischen Chemie, Band 4, Seite 105, 108 und 109, Urban und Schwarzenberg, 3. Auflage, Berlin-München, 1953), zur Herstellung von Polycarbonatkunststoffen, Pflanzenschutzmitteln und zur Herstellung von Bakteriziden (DE-A-21 31 555, DE-A-12 13 419 und Ullmanns Enzyklopädie der technischen Chemie, 4. Auflage, Band 9, Seite 383, Verlag Chemie 1975).

### Beispiele

Zu der in Tabelle 1 angegebene Menge des Katalysators und 9 g (100 mmol) Phenol wurden innerhalb von 10 Min. 9 g (100 mmol) Phosgen bei 110°C eingegast. Anschließend wurden im Verlauf der in Tabelle 1 angebenen Zeit 179 g (1900 mmol) Phenol und 198 g (2000 mmol) Phosgen zugefahren, 1 Stunde nachreagiert und das überschüssige Phosgen und der gelöste Chlorwasserstoff durch Stickstoffspülung entfernt. Nach Destillation erhielt man das Phenylchlorformiat, Sdp.: 94 bis 95°C/50 mbar. Das Destillat wurde gaschromatographisch untersucht.

| Beispiel | | | Reaktions dauer [h] | Ausbeute nach Destillation [%] | Zusammensetzung [GC-Flächen-%] | | Farbe [APHA] |
|---|---|---|---|---|---|---|---|
| | Katalysator | Konz. a) | | | Chlorbenzol | Phenylchlorformiat | |
| 1 | Dimethylformamid | 2 | 5 | 91 | < 0,1 | 99,7 | < 150 |
| 2 | Tetramethylharnstoff | 2 | 7 | 92 | < 0,1 | 99,6 | < 10 |
| 3 | Tetrabutylharnstoff | 2 | 5 | 94 | 0,1 | 99,6 | 30 |
| 4 | Triphenylphosphin | 2 | 4 | 92 | 0,5 | 99,3 | 30 |
| 5 | Triphenylphosphit | 2 | 3 | 95 | 0,7 | 99,1 | < 10 |
| 6 | Dimethylpropylenharnstoff | 2 | 3 | 96 | - | 99,9 | < 10 |
| 7 | Rückstand Beispiel 2 | - | 9 | 94 | < 0,1 | 99,5 | < 10 |
| 8 | Rückstand Beispiel 7 | - | nach 14 h | Reaktion unvollständig | - | - | - |
| 9 | Rückstand Beispiel 3 | - | nach 14 h | Reaktion unvollständig | - | - | - |
| 10 | Rückstand Beispiel 5 | - | 8 | 94 | 1 | 98,8 | < 10 |
| 11 | Rückstand Beispiel 10 | - | nach 14 h | Reaktion unvollständig | - | - | - |
| 12 | Rückstand Beispiel 6 | - | 3,5 | 96 | - | 99,9 | < 10 |
| 13 | Rückstand Beispiel 12 | - | 4,5 | 97 | - | 99,9 | < 10 |
| 14 | Dimethylpropylenharnstoff | 1 | 3,5 | 94 | - | 99,9 | < 10 |
| 15 | Rückstand Beispiel 14 | - | 7 | 94 | - | 99,9 | < 10 |
| 16 | Dimethylpropylenharnstoff | 0,5 | 5,5 | 97 | - | 99,9 | < 10 |
| 17 | Rückstand Beispiel 16 | - | nach 14 h | Reaktion unvollständig | - | - | - |

| | | | | | | | |
|---|---|---|---|---|---|---|---|
| a) Konzentration in Mol-% bezogen auf das Phenol II | | | | | | | |

## Patentansprüche

1. Verfahren zur Herstellung von Chlorameisensäurearylestern der allgemeinen Formel I 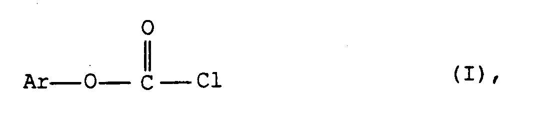 in der
Ar ein gegebenenfalls ein- bis fünffach durch C₁- bis C₆-Alkyl, C₂- bis C₆-Alkenyl, C₃- bis C₈-Cycloalkyl, C₄- bis C₁₀-Cycloalkyl-alkyl, C₁- bis C₆-Halogenalkyl, C₁-bis C₆-Alkoxy, C₁- bis C₄-Alkylthio, Halogen, Cyano, C₂- bis C₆-Alkylcarbonyloxy, Formyl, C₂- bis C₆-Dialkylamino, Aryl, Aryloxy, Arylthio, Aroyl, C₇- bis C₁₀-Aralkyl, C₇- bis C₁₀-Aralkoxy, Arylsulfonyl und/oder C₇- bis C₁₀-Aralkylthio und/oder ein- bis zweifach durch Chlorformyl und/oder Nitro substituiertes Aryl
bedeutet, durch Umsetzung von Phosgen und einem Phenol der allgemeinen Formel II
Ar―OH (II)
, in der Ar die obengenannten Bedeutungen hat, in Gegenwart einer stickstoffenthaltenden Verbindung bei Temperaturen von 60 bis 180°C und Drücken von 0,01 bis 5 bar, dadurch gekennzeichnet, daß man als stickstoffenthaltende Verbindung einen cyclischen Harnstoff der allgemeinen Formel III 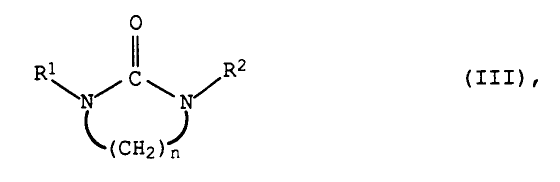 in der R¹ und R² für C₁- bis C₄-Alkyl und n für 2 oder 3 stehen, einsetzt.

2. Verfahren zur Herstellung von Chlorameisensäurearylestern nach Anspruch 1, dadurch gekennzeichnet, daß man als cyclischen Harnstoff III N,N'-Dimethylpropylen- oder N,N'-Dimethylethylenharnstoff einsetzt.

3. Verfahren zur Herstellung von Chlorameisensäurearylestern nach den Ansprüchen 1 bis 2, dadurch gekennzeichnet, daß das Molverhältnis von cyclischem Harnstoff III zum Phenol II 0,001:1 bis 0,2:1 beträgt.

4. Verfahren zur Herstellung von Chlorameisensäurearylestern nach den Ansprüchen 1 bis 3, dadurch gekennzeichnet, daß man die Umsetzung bei Temperaturen von 80 bis 160°C durchführt.

5. Verfahren zur Herstellung von Chlorameisensäurearylestern nach den Ansprüchen 1 bis 4, dadurch gekennzeichnet, daß man die Umsetzung bei Normaldruck durchführt.

## Claims

1. A process for preparing aryl chloroformates of the general formula I 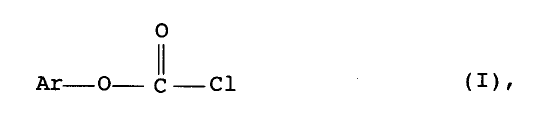 where
Ar is aryl which is unsubstituted or substituted once to five times by C₁-C₆-alkyl, C₂-C₆-alkenyl, C₃-C₈-cycloalkyl, C₄-C₁₀-cycloalkylalkyl, C₁-C₆-haloalkyl, C₁-C₆-alkoxy, C₁-C₄-alkylthio, halogen, cyano, C₂-C₆-alkyl-carbonyloxy, formyl, C₂-C₆-dialkylamino, aryl, aryloxy, arylthio, aroyl, C₇-C₁₀-aralkyl, C₇-C₁₀-aralkoxy, arylsulfonyl and/or C₇-C₁₀-aralkylthio and/or once or twice by chloroformyl and/or nitro
by reacting phosgene and a phenol of the general formula II
Ar―OH (II)
, where Ar has the abovementioned meanings, in the presence of a nitrogen-containing compound at from 60 to 180°C under from 0.01 to 5 bar, wherein the nitrogen-containing compound used is a cyclic urea of the general formula III 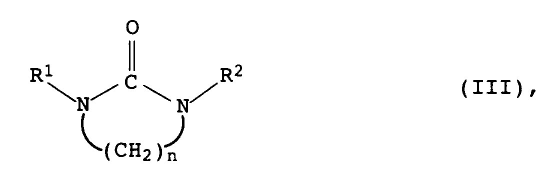 where R¹ and R² are each C₁-C₄-alkyl and n is 2 or 3.

2. A process for preparing aryl chloroformates as claimed in claim 1, wherein N,N'-dimethylpropylene- or N,N'-dimethylethyleneurea is used as cyclic urea III.

3. A process for preparing aryl chloroformates as claimed in claims 1 and 2, wherein the molar ratio of cyclic urea III to phenol II is from 0.001:1 to 0.2:1.

4. A process for preparing aryl chloroformates as claimed in any of claims 1 to 3, wherein the reaction is carried out at from 80 to 160°C.

5. A process for preparing aryl chloroformates as claimed in any of claims 1 to 4, wherein the reaction is carried out under atmospheric pressure.

## Revendications

1. Procédé de préparation d'esters aryliques de l'acide chloroformique de la formule générale I 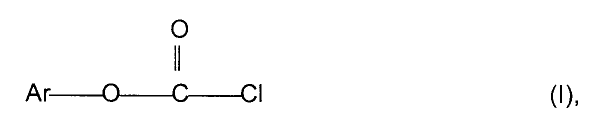 dans laquelle
Ar représente un radical aryle éventuellement substitué de une à cinq fois par des radicaux alkyle en C₁ à C₆, alcényle en C₂ à C₆, cycloalkyle en C₃ à C₈, cycloalkyl(C₄ à C₁₀)alkyle, halogénoalkyle en C₁ à C₆, alcoxy en C₁ à C₆, alkylthio en C₁ à C₄, des atomes d'halogènes, des radicaux cyano, alkyl(C₂ à C₆)carbonyloxy, formyle, dialkylamino en C₂ à C₆, aryle, aryloxy, arylthio, aroyle, aralkyle en C₇ à C₁₀, aralcoxy en C₇ à C₁₀, arylsulfonyle et/ou aralkylthio en C₇ à C₁₀ et/ou une ou deux fois par des radicaux chloroformyle et/ou nitro,
par la réaction du phosgène et d'un phénol de la formule générale II
Ar―OH (II)
, dans laquelle Ar possède les significations qui lui ont été attribuées ci-dessus, en présence d'un composé contenant de l'azote, à des températures de 60 à 180°C et sous des pressions de 0,01 à 5 bars, caractérisé en ce que, à titre de composé contenant de l'azote, on utilise une urée cyclique de la formule générale III 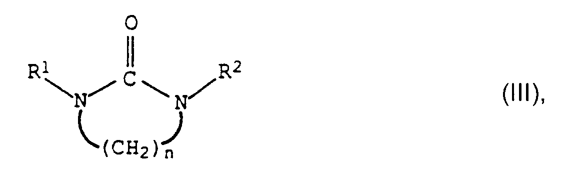 dans laquelle R¹ et R² représentent des radicaux alkyle en C₁ à C₄ et n est égal à 2 ou 3.

2. Procédé de préparation d'esters aryliques de l'acide chloroformique suivant la revendication 1, caractérisé en ce que l'on utilise, à titre durée cyclique III, la N,N'-diméthylpropylène- ou N,N'-diméthyléthylèneurée.

3. Procédé de préparation d'esters aryliques de l'acide chloroformique suivant l'une quelconque des revendications 1 et 2, caractérisé en ce que le rapport molaire de l'urée cyclique III au phénol II varie de 0,001:1 à 0,2:1.

4. Procédé de préparation d'esters aryliques de l'acide chloroformique suivant l'une quelconque des revendications 1 à 3, caractérisé en ce que l'on entreprend la réaction à des températures de 80 à 160°C.

5. Procédé de préparation d'esters aryliques de l'acide chloroformique suivant l'une quelconque des revendications 1 à 4, caractérisé en ce que l'on entreprend la réaction à la pression normale.
